# EUROPÄISCHE PATENTSCHRIFT

(11) **EP 3 919 107 B1**
(45) Veröffentlichungstag und Bekanntmachung des Hinweises auf die Patenterteilung: **07.08.2024**
(21) Anmeldenummer: 21177389.0
(22) Anmeldetag: 02.06.2021
(51) Int. Cl.: A61M 16/00, A63B 23/18

(54) **ATEMTHERAPIEGERÄT**
RESPIRATORY TREATMENT DEVICE
APPAREIL DE THÉRAPIE RESPIRATOIRE

(30) Priorität: 02.06.2020 DE 102020114617
(43) Veröffentlichungstag der Anmeldung: 08.12.2021
(73) Patentinhaber: Bittigkoffer, Peter, 76831 Billigheim (DE)
(72) Erfinder: Bittigkoffer, Peter, 76831 Billigheim (DE)
(74) Vertreter: Patentanwälte Dr. Keller, Schwertfeger Partnerschaft mbB

(56) Entgegenhaltungen:
- EP-A1- 3 156 093
- US-A1- 2011 146 672
- US-A1- 2016 375 213
- US-A1- 2019 105 534

## Beschreibung

### Technisches Gebiet:

Die vorliegende Erfindung betrifft ein Atemtherapiegerät, welches insbesondere für die Behandlung chronischer obstruktiver Atemwegserkrankungen bzw. Ventilationsstörungen geeignet ist.

### Stand der Technik:

Chronische obstruktive Atemwegserkrankungen bzw. Ventilationsstörungen weisen eine komplexe Pathophysiologie auf und sind insbesondere durch chronische Entzündungen, erhöhte Schleimsekretion sowie Verengungen der Atemwege (Atemwegs- bzw. Bronchialobstruktion) gekennzeichnet. Ein zentrales Merkmal dieser Erkrankungen ist die Instabilität der kleineren Atemwege mit Kollapsneigung während der Ausatmung als Folge der Erkrankung. Der Bronchialkollaps resultiert in einer Limitation des sogenannten exspiratorischen Flusses, welches zum Phänomen der Lungenüberblähung (Hyperinflation) führt. Diese ist durch ein erhöhtes Gasvolumen in der Lunge und den Atemwegen am Ende der spontanen Exspiration (Ausatmung) charakterisiert und kann mit Kurzatmigkeit bzw. Atemnot (Dyspnoe) einhergehen.

Patienten mit chronisch obstruktiver Ventilationsstörung haben dementsprechend Schwierigkeiten, die sich in der Lunge befindliche Atemluft wieder auszuatmen, da durch das Zusammenfallen der kleineren Bronchien (Bronchiolen) die verbrauchte Luft nicht mehr aus den distalen Abschnitten der Lunge bzw. den Lungenalveolen herausgeführt werden kann und dort gefangen bleibt (air trapping). Neben der Überblähung der Lunge kommt es zu einer Störung des Gasaustauschs, da die verbrauchte Luft in den Lungenalveolen, an denen der Austausch von Sauerstoff und Kohlenstoffdioxid erfolgt, nicht am Gasaustausch teilnimmt und zudem weniger frische Luft bei der Einatmung einströmen kann (Senkung der inspiratorischen Kapazität). Die Folge der akuten und zunächst reversiblen Lungenüberblähung können Kurzatmigkeit bzw. Atemnot sein, insbesondere unter Belastung (Belastungsdyspnoe). Die Atemnot bei Belastung induziert beim Patienten ein Vermeidungsverhalten, so dass er sich weniger körperlich belastet. Aus der Inaktivität resultiert schnell ein "Teufelskreis", der mit einer Verschlechterung der Erkrankung und der Lebensqualität einhergeht.

Die Erkrankungen sind durch einen progredienten Verlauf gekennzeichnet, wobei die Lungenüberblähung mit einhergehender Dyspnoe eine wichtige Rolle bei der Pathophysiologie bzw. Progression der Erkrankungen spielt. Neben Exazerbationen ist die Lungenüberblähung bzw. die begleitende Dyspnoe die Hauptursache für die Verschlechterung der Lebensqualität und die zunehmende Einschränkung der körperlichen Belastungstoleranz. Wiederholen sich die Überblähungen durch die unvollständige Ausatmung über längere Zeit oder Jahre hinweg, kann es zur irreversiblen Schädigung des Lungengewebes mit Zerstörung und Überdehnung der Lungenbläschen (Alveolen) kommen. Die Folge ist die Entstehung eines chronischen Lungenemphysems mit permanenter chronischer Lungenüberblähung und Atemnot auch in Ruhe.

Die hinsichtlich ihrer Prävalenz bedeutsamsten und bekanntesten Vertreter chronischer obstruktiver Atemwegserkrankungen sind die chronisch obstruktive Lungenerkrankung (englisch: "chronic obstructive pulmonary disease", Abkürzung: COPD) sowie Asthma bronchiale (oder kurz Asthma). Da sich die Definition der Erkrankung COPD aus der Feststellung einer chronischen Bronchitis plus einer dauerhaften Verengung der unteren Atemwege mit und ohne Lungenemphysem herleitet, werden auch das Lungenemphysem sowie die chronisch obstruktive Bronchitis unter dem Begriff COPD zusammengefasst.

Das Behandlungsziel in der Therapie der Erkrankungen besteht darin, das Fortschreiten der Erkrankung zu mindern oder aufzuhalten und die Lebensqualität der Patienten zu verbessern. Die Vermeidung bzw. die Reduktion der dynamischen Lungenüberblähung unter Belastung und somit die Verminderung der Dyspnoe stellt, neben der Besserung der körperlichen Leistungsfähigkeit, ein wichtiges zentrales Element der Therapie dar, um die Progression der Erkrankung und insbesondere die Ausbildung einer dauerhaften, irreversiblen Lungenüberblähung bzw. eines Lungenemphysems zu vermeiden.

Die pharmakotherapeutische Behandlung der chronisch obstruktiven Atemwegserkrankungen erfolgt überwiegend mit inhalierbaren Medikamenten (Bronchodilatoren), die als Dosieraerosole oder Pulver mit Hilfe von Inhalatoren verabreicht werden. Neben der Vermeidung von Risikofaktoren spielen auch körperliches Training sowie physiotherapeutische Atemtherapie eine wichtige Rolle.

Als therapeutische Hilfsmittel für die Atemtherapie werden in der klinischen Praxis sogenannte PEP (positive expiratory pressure)-Systeme bzw. Atemtherapiegeräte eingesetzt. Mit Hilfe der PEP-Systeme entsteht beim Ausatmen in die entsprechenden Atemtherapiegeräte ein positiver Ausatemdruck (PEP), der sich bis in die tiefen Bronchien fortsetzt. Damit sind die Therapiegeräte in der Lage, das Kollabieren der Atemwege zu verhindern, so dass die verbrauchte Luft aus der Lunge herausströmen kann.

Die meisten auf dem Markt befindlichen Geräte zielen dabei insbesondere auf die Mobilisation von festsitzendem Bronchialsekret ab und sorgen, entsprechend ihrer Bauweise als oszillierende PEP (OPEP)-Systeme, für eine Oszillation (Schwingung) des PEP und der Ausatemluft, was die Sekretelimination unterstützt.

Die WO 2014/154541 A1 beschreibt beispielsweise ein solches Therapiegerät bzw. OPEP-System zur Behandlung von Atemwegserkrankungen. Bei diesem Therapiegerät ist an einem Mundstück mit einem Durchlasskanal ein Schlauch aus einem biegeelastischen Werkstoff befestigt, so dass die Atemluft in den Schlauch gepresst werden kann. Weiterhin ist ein gekrümmter oder gebogener Auflagekörper vorgesehen, der fest mit dem Mundstück verbunden ist und auf dem der Schlauch angeordnet ist. Beim Ausatmen strömt die Atemluft durch den Durchlasskanal in den Schlauch und versetzt diesen beim Ausströmen in Schwingungen.

Auch aus der DE 10 2015 214 141 A1 ist ein entsprechendes Atemtherapiegerät bekannt. Dieses Atemtherapiegerät umfasst einen Atemluftkanal mit einer Verengung mit veränderbarem Durchlassquerschnitt, einen Drucksensor, der ausgebildet ist, den Wert eines im Atemluftkanal herrschenden Drucks zu erfassen, und eine Stellvorrichtung, die dazu ausgebildet und angeordnet ist, den Durchlassquerschnitt der Verengung zu verändern.

Des Weiteren zielen sie auf ein Training der Lungenfunktion und der Atemmuskulatur ab. Dementsprechend erzeugen sie relativ hohe positive Ausatemdrücke bei der Anwendung.

Optional können einige Therapiegeräte auf dem Markt in Kombination mit Verneblern zur Inhalation eingesetzt werden.

Der Nachteil der bekannten Atemtherapiegeräte nach dem PEP-System ist, dass diese häufig sehr voluminös und sperrig sind. Dies ist vor allem dann nachteilig, wenn der Patient unterwegs ist. Der Patient wäre häufig gezwungen, nur wegen des Atemtherapiegeräts permanent eine Tasche mit sich zu führen, wenn er das Atemtherapiegerät im Notfall zur Hand haben möchte.

Da die Geräte zudem aus mehreren Einzelteilen zusammengefügt sind und zur Reinigung auseinandergebaut werden müssen, bergen sie das Risiko der Fehlfunktion nach falschem Zusammenbau nach Reinigung. Auch das Vorhandensein von verschiedenen Einstelloptionen für den PEP erschwert den intuitiven Einsatz im Notfall. In Notsituationen sind insbesondere demente Personen mit dem Gebrauch dieser Atemtherapiegeräte überfordert.

Zudem sind die bekannten Therapiegeräte eher auf eine passagere, zeitlich limitierte Anwendung ausgerichtet und erzeugen aufgrund ihrer Zielfunktion relativ hohe PEPs, die im Gebrauch für den Patienten anstrengend sind und keine längere Anwendung, insbesondere nicht unter Belastung, ermöglichen.

In Ruhe kommt es erst bei weit fortgeschrittener Erkrankung zur Lungenüberblähung mit eingehender Atemnot. Das pathologische Phänomen der Überblähung der Lunge mit der Folge irreversibler Schädigung des Lungengewebes erfolgt vielmehr hauptsächlich in alltäglichen Belastungssituationen, z.B. beim Treppensteigen, beim schweren Tragen oder einfach nur Tätigkeiten im Haushalt oder beim Gehen. Im Hinblick auf die Pathophysiologie und Progression der Erkrankung ist daher die Vermeidung bzw. Reduktion der Lungenüberblähung in Belastungssituationen von zentraler Bedeutung. Bei der Prävention der irreversiblen Schädigung der Lunge durch dynamische Überblähung geht es vor allem um diese Belastungssituationen im Alltag, bei denen ohne Therapiegerät nicht richtig bzw. nicht vollständig ausgeatmet wird.

Im Rahmen der physiotherapeutischen Atemtherapie wird die exspiratorische Atemtechnik der sogenannten Lippenbremse erlernt, mit Hilfe derer der Patient ohne Zuhilfenahme eines Atemtherapiegeräts einen PEP erzeugen kann. Für die Lippenbremse werden die Lippen leicht aufeinandergelegt und durch einen kleinen Spalt zwischen den leicht gespitzten Lippen langsam gegen den erhöhten Widerstand ausgeatmet. Die notwendige Konzentration und Kraftanstrengung des Patienten zum Ausbilden der Technik ist allerdings nicht unerheblich, insbesondere bei Belastung. Daher kann die Technik oftmals vom Patienten nur kurzzeitig umgesetzt werden. Bei Atemnot ist es für den Patienten noch schwieriger die Technik anzuwenden. Dieses steht dem Erfolg einer längeren und häufigen Anwendung der Technik unter Belastung sowie dem korrekten Einsatz im Notfall entgegen.

Aus dem Dokument US2016/0375213 A1 ist ein Atemtherapiegerät zur Stressreduzierung und Vermeidung von Hyperventilation bekannt. Aus dem Dokument US2019/0105534 A1 ist eine Stimmtrainingsvorrichtung zur akuten Stimmtherapie oder zur fortlaufenden Erhaltung der Stimmgesundheit bekannt.

### Darstellung der Erfindung:

Es ist daher die Aufgabe der vorliegenden Erfindung, ein Atemtherapiegerät bzw. eine Ausatemhilfe bereitzustellen, welches/welche der Patient einfach und ständig mit sich führen kann, um dieses/diese jederzeit in Belastungssituationen im Alltag oder im Fall eines Anfalls mit Atemnot schnell, einfach und intuitiv einsetzen zu können Dabei sollte die Anwendung des Atemtherapiegeräts keine zusätzliche Kraftanstrengung für den Patienten bedeuten und somit eine längere Anwendung - auch mehrmals täglich - ermöglichen.

Erfindungsgemäß wird die Aufgabe durch ein Atemtherapiegerät gemäß Anspruch 1 gelöst. Das Atemtherapiegerät umfasst ein Therapieelement und eine Halterung, wobei das Therapieelement einen länglichen Grundkörper mit einem ersten Endabschnitt und einem zweiten Endabschnitt, die entgegengesetzt zueinander angeordnet sind, sowie einen mittleren Abschnitt umfasst, wobei ein Mundstück am ersten Endabschnitt vorgesehen ist, wobei der Grundkörper eine erste Öffnung an der Stirnseite des ersten Endabschnitts und eine zweite Öffnung an der Stirnseite des zweiten Endabschnitts aufweist und im Inneren des Grundkörpers ein Strömungskanal vorgesehen ist, der sich von der ersten Öffnung an der ersten Stirnseite bis zur zweiten Öffnung an der zweiten Stirnseite erstreckt, wobei an dem zweiten Endabschnitt ein Mundstück vorgesehen ist, wobei die Halterung an der Außenseite des Grundkörpers, vorzugweise im Bereich des mittleren Abschnitts vorgesehen ist, und das Mundstück am ersten Endabschnitt trichterförmig ausgebildet ist, wobei sich der Außendurchmesser des Mundstücks am ersten Endabschnitt von der ersten Öffnung in Richtung des mittleren Abschnitts erweitert.

Das Therapieelement des erfindungsgemäßen Atemtherapiegeräts ermöglicht es dem Patienten, mit Hilfe des PEP-Prinzips die Überblähung sowohl in der Alltagsbelastung des Patienten als auch im Falle eines Anfalls mit Atemnot zu minimieren. Die direkt am Grundkörper vorgesehene Halterung, die vorzugweise im Bereich des mittleren Abschnitts, aber auch an dem zweiten Endabschnitt, an dem sich kein Mundstück befindet, vorgesehen sein kann, sorgt dafür, dass der Patient das Therapieelement an einem Bekleidungsstück befestigen kann oder an einem Körperteil wie etwa am Arm oder am Hals tragen kann, so dass das Therapieelement immer zur Verfügung steht. Um den Patienten während belastender Situationen vor der Überblähung und deren negativen Folgen zu schützen, kann das Atemtherapiegerät somit ohne zusätzliche Kraftanstrengung über längere Zeiträume über den gesamten Tag angewendet und als ständiger Begleiter im Alltag integriert werden.

Hierbei hat es sich als vorteilhaft erwiesen, dass das Mundstück einstückig mit dem Grundkörper verbunden ist. Es kann somit nicht verloren gehen. Darüber hinaus werden Fehlfunktionen vermieden, da das Atemtherapiegerät zum einen intuitiv einsetzbar ist und zum anderen das Risiko des falschen Zusammenbaus nach Reinigung minimiert ist. Des Weiteren besteht kein Verschleiß oder eine Materialermüdung im Bereich von Verbindungsstellen für den Zusammenbau, so dass eine dadurch bedingte Fehlfunktion vermieden wird.

Bei einer besonders bevorzugten Ausführungsform sind der Grundkörper, das Mundstück und die Halterung einstückig ausgebildet, so dass ein fehlerhafter Zusammenbau ausgeschlossen werden kann. Ein solches einstückiges Therapieelement hat zudem den Vorteil der größeren Robustheit bei Herunterfallen. Um gegebenenfalls die Mundstücke austauschen zu können, sind bei einer alternativen Ausführungsform der Grundkörper und die Halterung einstückig ausgebildet.

Erfindungsgemäß ist das Mundstück trichterförmig ausgebildet, wobei sich der Außendurchmesser des Mundstücks von der ersten Öffnung in Richtung des mittleren Abschnitts erweitert. Idealerweise ist der Öffnungswinkel des Trichters kleiner als 30°, vorzugsweise kleiner als 10°. Hierdurch ist es möglich, dass der Patient sogenannte spitze Lippen bekommt, wenn er in das Mundstück bläst bzw. ausatmet. Diese Art des Ausatmens trägt dazu bei, dass das Kollabieren der Atemwege, insbesondere der Bronchien, verhindert wird.

Bei einer alternativen Ausführungsform, bei der auch die Technik der spitzen Lippen ohne zusätzliche Kraftanstrengung umgesetzt werden kann, weist das Mundstück eine Ober- und Unterseite mit jeweils einer Seitenbreite und einer Seitenlänge auf, wobei der Abstand zwischen der Ober- und Unterseite geringer als die Seitenbreite der Ober- und/oder der Unterseite ist. Vorzugsweise ist die Seitenbreite der Ober- und/oder der Unterseite mindestens doppelt so groß wie der Abstand zwischen der Ober- und Unterseite.

Die Ober- und/oder Unterseite können als ebene oder gekrümmte Fläche ausgebildet sein. Dabei können beide Seiten als ebene Fläche bzw. beide Seiten als gekrümmte Fläche ausgebildet sein. So kann das Mundstück beispielsweise die Form eines Entenschnabels oder einer Zigarettenspitze aufweisen, um angenehm im Mund gehalten zu werden. Alternativ kann die eine Seite als ebene und die andere Seite als gekrümmte Fläche ausgebildet sein, wie beispielsweise beim Mundstück einer Trillerpfeife oder Blockflöte.

Um das Mundstück sicher im Mund halten zu können, ist es von Vorteil, dass die Oberfläche des Mundstücks zumindest abschnittsweise uneben, insbesondere stufig ist, wobei vorzugsweise bei einer Stufe mit einer Stufenkante die Stufenkante in Richtung des mittleren Abschnitts ausgerichtet ist. Selbst bei einem glatten Oberflächenmaterial können die Stufen der Mundstücke für einen sicheren Halt des Atemtherapiegeräts sorgen.

Zusätzlich oder alternativ kann auf der Außenseite des Mundstücks wenigstens abschnittsweise eine rutschfeste Materialschicht vorgesehen ist. Ein Beispiel für eine geeignete rutschfeste Materialschicht ist z.B. Silikon. Durch die Wahl eines Silikons mit geringem Härtegrad kann eine weiche, anpassungsfähige und damit angenehme Oberfläche für den Lippen- bzw. Mundkontakt erzeugt werden. Die Mundstücke können hierbei lediglich partiell weichere Silikonoberflächen aufweisen, die z.B. als ring- bzw. wulstförmige oder noppenartige Ausstülpungen bzw. Erhebungen auf einer ansonsten harten Oberfläche verteilt sind.

Es versteht sich, dass sowohl das gesamte Therapieelement als auch lediglich die Mundstücke bzw. Teile der Mundstücke eine derartige rutschfeste Materialschicht, insbesondere eine Silikonoberfläche aufweisen können.

Weiterhin ist bei einer bevorzugten Ausführungsform vorgesehen, dass das Mundstück wenigstens eine Vertiefung, insbesondere wenigstens eine Mulde und/oder wenigstens eine Erhöhung, insbesondere wenigstens eine Rippe, an seiner Außenseite aufweist. Selbst bei einem glatten Oberflächenmaterial des Mundstücks kann die wenigstens eine Vertiefung oder die wenigstens eine Erhöhung des Mundstücks für einen sicheren Halt des Atemtherapiegeräts sorgen.

Da das Atemtherapiegerät für den Dauereinsatz ausgelegt ist, ist es aus hygienischen Gründen von Vorteil, dass das Therapieelement, insbesondere das Mundstück vorzugsweise aus einem speichelfesten sowie unbedenklichen Material, insbesondere aus einem Kunststoff einschließlich Silikon hergestellt ist.

Bei einem Atemtherapiegerät, welches gemäß dem PEP-Verfahren arbeitet, ist die Erzeugung eines positiven Drucks bzw. eines Gegendrucks beim Ausatmen (positiver Ausatemdruck) in das Atemtherapiegerät notwendig. Der für den Patienten benötigte positive Ausatemdruck lässt sich beispielsweise mit Hilfe der Geometrie des Strömungskanals einstellen.

Unabhängig von der Oberflächenbeschaffenheit der Mundstücke ist daher darauf zu achten, dass die Form bzw. die Ausmaße des Strömungskanals während des Gebrauchs unverändert bleiben. Bei einer bevorzugten Ausführungsform besteht der Grundkörper aus einem festen, formstabilen Material, wie etwa einem Kunststoff.

Weiterhin ist es von Vorteil, dass das Atemtherapiegerät, insbesondere das Therapieelement aus einem hitzebeständigen Kunststoff bzw. Silikon hergestellt ist, da es sich hierdurch leicht reinigen bzw. desinfizieren lässt.

Gemäß einer bevorzugten Ausführungsform wird der positive Ausatemdruck bei einem Atemtherapiegerät auf einfache Weise dadurch hergestellt, dass sich der Strömungskanal von der ersten Öffnung zum mittleren Abschnitt erweitert und sich vom mittleren Abschnitt zur zweiten Öffnung verengt. Als besonders vorteilhaft hat es sich erwiesen, dass sich der Strömungskanal von der ersten Öffnung zum mittleren Abschnitt konisch erweitert und sich vom mittleren Abschnitt zur zweiten Öffnung konisch verengt.

Bei einer alternativen Ausführungsform weist der Strömungskanal im Bereich des ersten und/oder im Bereich des zweiten Endabschnitts einen konstanten Durchmesser auf. Der dadurch erzeugbare positive Ausatemdruck kann für einzelne Patienten bereits ausreichend sein, um ein Kollabieren der Atemwege zu verhindern.

Bei einer bevorzugten Weiterbildung ist der Durchmesser des Strömungskanals konstant.

Erfindungsgemäß ist an dem zweiten Endabschnitt ein Mundstück vorgesehen. Dies hat den Vorteil, dass der Patient im Falle eines Atemnotanfalls nicht überlegen muss, welche Seite das Mundstück bzw. welche Seite das Endstück des Therapieelements ist.

Insbesondere bei Therapieelementen, bei denen an beiden Endabschnitten ein Mundstück vorgesehen ist, ist es von Vorteil, dass die Halterung mittig zwischen den beiden Endabschnitten angeordnet ist. Dies hat den Vorteil, dass das Atemtherapiegerät sicher mit einem Befestigungsmittel wie etwa einem Band, einer Kette oder einer Schlaufe verbunden werden kann und die Mundstücke an beiden Endabschnitten ohne ein störendes Befestigungsmittel verwendet werden können.

Es ist weiterhin von Vorteil, dass am ersten Endabschnitt und am zweiten Endabschnitt jeweils ein Mundstück vorgesehen ist, wobei vorzugsweise der erste Endabschnitt und der zweite Endabschnitt spiegelsymmetrisch zum mittleren Abschnitt ausgebildet sind. Somit kann der Patient beide Mundstücke gleichwertig benutzen.

Es hat sich herausgestellt, dass ein besonders gutes Therapieergebnis dadurch erreicht wird, dass der Durchmesser des Strömungskanals im Bereich von 2 mm bis 8 mm, vorzugsweise im Bereich von 3 mm bis 7 mm liegt. Bei diesen Durchmessern des Strömungskanals lässt sich der für das PEP-Prinzip benötigte positive Ausatemdruck für den Patienten einfach und effektiv aufbauen.

Weiterhin hat es sich als vorteilhaft erwiesen, dass die Länge des Strömungskanals etwa 3 cm bis 7 cm, vorzugsweise 4 cm bis 6 cm beträgt. Diese Länge ist ausreichend, um eine Ausatmung mit Gegendruck beim Ausatmen (positiver Ausatemdruck) zu bewirken. Die kurze Länge ermöglicht es dem Patienten, das Atemtherapiegerät unauffällig um den Hals, am Arm oder an der Bekleidung zu tragen.

Mit Hilfe der unterschiedlichen Durchmesser und Längen des Strömungskanals können beispielsweise Größenunterschiede der Patienten berücksichtigt werden. So benötigen Kinder andere Größen des Therapieelements als Erwachsene.

Es ist von Vorteil, dass das Atemtherapiegerät so ausgelegt ist, dass der positive Ausatemdruck (PEP) beim langsamen, gleichmäßigen Ausatmen in das Therapiegerät kleiner gleich 10 mbar ist, vorzugsweise im Bereich von 5 mbar bis 1 mbar, idealerweise im Bereich von 3 mbar bis 1 mbar liegt. Der Patient wird auch bei längeren Anwendungen des Therapiegerätes nicht übermäßig belastet, wohingegen bei der Technik der spitzen Lippen bzw. der Lippenbremse ohne Therapiegerät dieses Ausatemmanöver nicht lange durchzuhalten ist. Hierdurch kann das Atemtherapiegerät nicht nur für Atemtrainingsübungen, sondern auch im Alltag eingesetzt werden. Der Patient wird auch bei längeren Anwendungen nicht übermäßig belastet. Der häufigere Gebrauch des Atemtherapiegeräts trägt zur Ausbremsung der Progression der Erkrankung bei.

Um ein Befestigungsmittel wie etwa ein Band sicher an dem Atemtherapiegerät zu befestigen, ist es von Vorteil, dass die Halterung einen Durchgang oder ein Befestigungselement für ein Befestigungsmittel umfasst.

Bei einer bevorzugten Ausführungsform ist ein Band vorgesehen, wobei das Band an der Halterung befestigt ist. Das Band kann unmittelbar an der Halterung, beispielsweise mittels einer Schlaufe oder eines Knotens oder mittels eines weiteren Befestigungselements wie etwa eines Hakens, insbesondere eines Karabinerhakens an der Halterung befestigt sein. Anstelle des Hakens können auch andere lösbare Verbindungen, beispielsweise in Form von Steckverbindungen vorgesehen sein.

Das Band kann als Halsband oder Armband ausgebildet sein, wobei vorzugweise das Band mit einem Sicherheitsverschluss versehen sein kann, um sich im Notfall unter Zug zu öffnen.

Zusätzlich oder alternativ können an dem Band wiederum Befestigungsmittel vorgesehen sein, um das Band beispielsweise an Kleidungsstücken zu befestigen. Um das Atemtherapiegerät schnell und einfach vom Band zu lösen und auch schnell wieder befestigen zu können, um z.B. das Atemtherapiegerät bei Anwendung vom Band abzutrennen, kann das Atemtherapiegerät mit Hilfe eines Steckverbindung am Band befestigt werden. Dazu muss am Band ein Element der Steckverbindung und am Atemtherapiegerät das andere Element der Steckverbindung verbunden sein, z.B. über Schlaufen.

Mit Hilfe des Bandes, welches beispielsweise um den Hals gelegt oder am Handgelenk angelegt wird, lässt sich das Atemtherapiegerät einfach transportieren. Das Therapieelement, das sehr klein und im Wesentlichen rohrförmig gestaltet werden kann, kann einfach und unauffällig an dem Band getragen werden und bei Bedarf auch unter der Kleidung versteckt werden.

Das Atemtherapiegerät weist somit insgesamt eine sehr geringe Größe und ein vergleichsweise geringes Gewicht auf, so dass der Patient das Atemtherapiegerät nicht festhalten muss, sondern lediglich mit dem Mund bzw. den Lippen halten kann. Somit hat der Patient bei Aktivitäten im Alltag beide Hände frei, z.B. um sich beim Treppensteigen am Handlauf festhalten zu können. Dieses ist im Hinblick auf die Sicherheit und die Therapietreue (Compliance) des Patienten von besonderer Bedeutung.

Das Atemtherapiegerät kann aufgrund seines geringen Gewichts auch im Liegen verwendet werden. In liegender Position ist das Atemtherapiegerät vorzugsweise über ein Band am Körper oder der Kleidung befestigt, um es zum einen nicht zu verlieren und zum anderen das ohnehin minimale Risiko einer Aspiration oder eines Verschluckens auszuschließen.

Wird das Atemtherapiegerät nicht benötigt, ist es aus hygienischen Gründen von Vorteil, dass eine Schutzhülle vorgesehen ist, die das Therapieelement wenigstens abschnittsweise umgibt. Vorzugsweise sind Belüftungsöffnungen vorgesehen, um eine Belüftung des Therapieelements insbesondere im Bereich des Mundstücks zu ermöglichen.

Das erfindungsgemäße Atemtherapiegerät ist insbesondere auf den Einsatz bei Patienten mit COPD und Asthma bronchiale ausgerichtet, jedoch nicht darauf beschränkt. Der Einsatz des erfindungsgemäßen Atemtherapiegeräts kann ebenfalls bei anderen chronischen obstruktiven Atemwegserkrankungen erfolgen, die mit dem Risiko einer Lungenüberblähung gekennzeichnet sind, wie z.B. eine spezielle Form des Lungenemphysems aufgrund eines vererbten Mangels des Enzyms Alpha-1-Antitrypsin, auch als Laurell-Eriksson-Syndrom oder AAT-Defizit bezeichnet.

### Kurze Beschreibung der Zeichnungen:

Bevorzugte Ausführungsformen werden anhand der beigefügten Zeichnungen näher erläutert, in denen zeigen:
- Fig. 1: ein Atemtherapiegerät gemäß einer ersten Ausführungsform in einer perspektivischen Ansicht,
- Fig. 2: das Therapieelement des in Fig. 1 dargestellten Atemtherapiegeräts in einer Seitenansicht mit einer Halterung gemäß einer ersten Ausführungsform,
- Fig. 3: einen Längsschnitt durch das in Fig. 2 dargestellte Therapieelement;
- Fig. 4: das Therapieelement des in Fig. 1 dargestellten Atemtherapiegeräts in einer Ansicht von oben mit einer Halterung gemäß einer zweiten Ausführungsform,
- Fig. 5: ein Atemtherapiegerät gemäß einer zweiten Ausführungsform in einer perspektivischen Ansicht,
- Fig. 6: einen Ausschnitt einer Seitenansicht des in Fig. 5 dargestellten Atemtherapiegeräts,
- Fig. 7: ein Therapieelement mit einer Halterung gemäß einer dritten Ausführungsform,
- Fig. 8: ein Therapieelement gemäß einer vierten Ausführungsform in einer perspektivischen Ansicht,
- Fig. 9: einen Längsschnitt durch das in Fig. 8 dargestellte Therapieelement;
- Fig. 10: ein Therapieelement gemäß einer fünften Ausführungsform in einer perspektivischen Ansicht,
- Fig. 11: ein Atemtherapiegerät gemäß einer weiteren Ausführungsform in einer perspektivischen Ansicht, und
- Fig. 12: einen Längsschnitt durch das in Fig. 11 dargestellte Therapieelement.

Wege zur Ausführung der Erfindung und gewerbliche Anwendbarkeit:

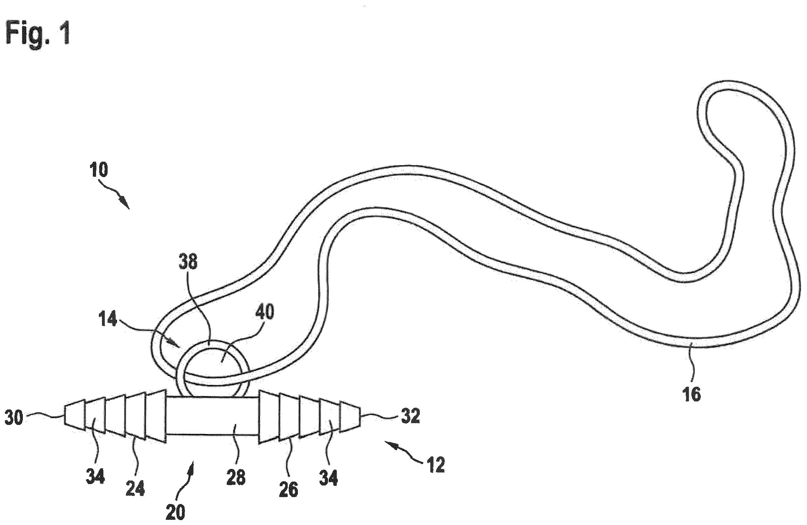

Fig. 1 zeigt ein Atemtherapiegerät 10 gemäß einer ersten Ausführungsform in einer perspektivischen Ansicht. Das Atemtherapiegerät 10 umfasst ein Therapieelement 12, eine Halterung 14 und ein Band 16. Das Therapieelement 12 und die Halterung 14 sind einstückig ausgebildet. Das Band 16 ist schnurartig mit einem vorzugsweise runden Querschnitt ausgebildet und lösbar an der Halterung 14 befestigt. Hierfür kann an dem Band 16 ein Verschluss oder dergleichen vorgesehen sein. Zusätzlich oder alternativ kann das Band 16 elastisch sein. Es versteht sich, dass das Band 16 auch jeden beliebigen anderen Querschnitt wie etwa eckig oder oval aufweisen kann.

Sowohl das Therapieelement 12 als auch die Halterung 14 und das Band 16 sind jeweils aus Kunststoff hergestellt, wobei insbesondere das Therapieelement 12 aus einem speichelechten, hitzebeständigen Kunststoff hergestellt ist. Alternativ kann das Band 16 aus einem textilen oder metallischen Material hergestellt sein. Auch das Therapieelement 12 und die Halterung 14 können aus einem anderen Material als Kunststoff hergestellt sein, wobei das für das Therapieelement 12 verwendete Material sowohl speichelecht als auch fest und formstabil sein sollte. Insbesondere ist das Therapieelement aus einem festen Silikonmaterial hergestellt.

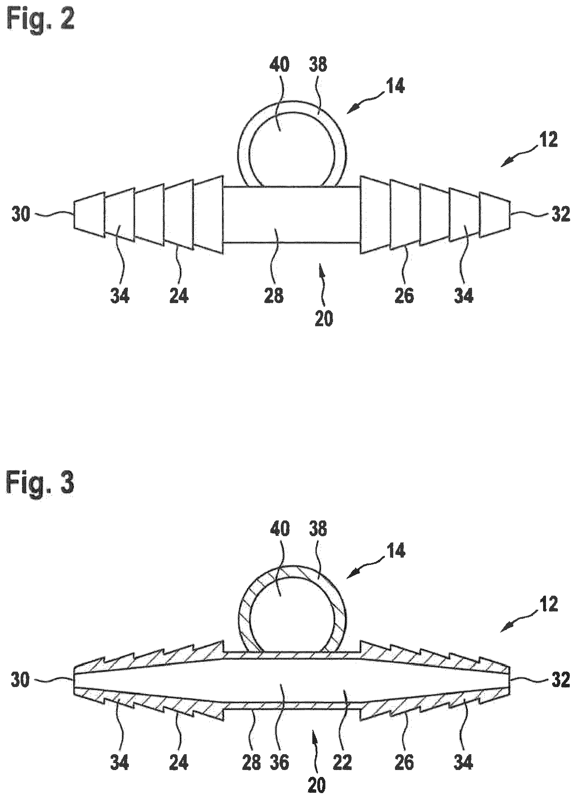

Wie insbesondere in den Figuren 2 und 3 zu erkennen ist, umfasst das Therapieelement 12 einen länglichen, rohrförmigen Grundkörper 20 mit einem Strömungskanal 22 im Inneren des Grundkörpers 20. Der Grundkörper 20 umfasst einen ersten Endabschnitt 24 und einen zweiten Endabschnitt 26, die entgegengesetzt zueinander angeordnet sind, sowie einen mittleren Abschnitt 28. An einer Stirnseite des ersten Endabschnitts 24 des Grundkörpers 20 ist eine erste Öffnung 30 vorgesehen. Eine zweite Öffnung 32 ist an der entgegengesetzt liegenden Stirnseite am zweiten Endabschnitt 26 angebracht. Der Strömungskanal 22 im Inneren des Grundkörpers 20 verläuft gradlinig und mündet jeweils in die erste Öffnung 30 bzw. zweite Öffnung 32.

Sowohl am ersten Endabschnitt 24 als auch am zweiten Endabschnitt 26 ist jeweils ein Mundstück 34 angebracht. Jedes Mundstück 34 ist einstückig mit dem Grundkörper 20 verbunden und trichterförmig ausgebildet, wobei sich der Außendurchmesser des Mundstücks 34 von der ersten Öffnung 30 bzw. von der zweiten Öffnung 32 in Richtung des mittleren Abschnitts 28 konisch erweitert.

Die Oberfläche des Mundstücks 34 ist stufig ausgebildet. Jede Stufe weist einen schrägen Stufenabschnitt und eine Stufenkante auf, wobei die Stufenkante in Richtung des mittleren Abschnitts 28 ausgerichtet ist.

Der Strömungskanal 22 erweitert sich von der ersten Öffnung 30 zum mittleren Abschnitt 28 und verengt sich wieder vom mittleren Abschnitt 28 zur zweiten Öffnung 32. Hierbei ist im mittleren Abschnitt 28 eine Strömungskammer 36 vorgesehen. Die Erweiterung des Strömungskanals 22 im ersten Endabschnitt 24 bzw. die Verengung des Strömungskanals 22 im zweiten Endabschnitt 26 erfolgt stetig, insbesondere konisch.

Wenn auch nicht dargestellt, so kann das Volumen der Strömungskammer 36 mittels entsprechender Verstellmittel verändert werden.

Im Bereich des ersten Endabschnitts 24 und im Bereich des zweiten Endabschnitts 26 weist der Strömungskanal 22 einen runden Querschnitt auf. Alternativ kann der Querschnitt des Strömungskanals 22 oval ausgebildet sein.

Die Halterung 14 ist an der Außenseite des Grundkörpers 20 im Bereich des mittleren Abschnitts 28 vorgesehen und an dem Grundkörper 20 befestigt. Im Detail ist die Halterung 14 selbst als Ring 38 oder Öse mit einem Durchgang 40 ausgebildet und mittig zwischen den beiden Mundstücken 34 im mittleren Abschnitt 28 des Grundkörpers 20 angebracht. Das Band 16 kann durch den Durchgang 40 hindurchgeführt werden.

Bei einer nicht dargestellten Ausführungsform umfasst die Halterung einen Befestigungssteg und eine Auskragung am freien Ende, so dass das Band an dem Befestigungssteg befestigt werden kann.

Der Durchmesser des Strömungskanals 22 außerhalb der Strömungskammer 36 liegt im Bereich von 2 bis 8 mm, vorzugsweise im Bereich von 3 bis 7 mm. Die Länge des Strömungskanals 22 beträgt etwa 3 bis 7 cm, vorzugsweise 4 bis 6 cm. Die Maße des Strömungskanals 22 sowie der Strömungskammer 36 sind so ausgelegt, dass bei der Anwendung ein positiver Ausatemdruck (PEP) im Bereich von 5 mbar bis 1 mbar erzielt wird.

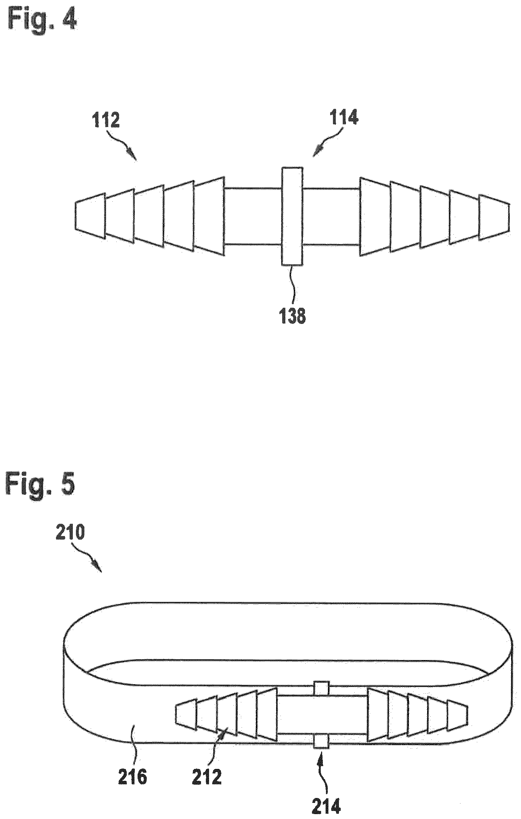

Fig. 4 zeigt eine alternative Ausführungsform des Therapieelements 112 mit einstückig verbundener Halterung 114, bei der der Ring 138 der Halterung 114 im Vergleich zu der in den Fig. 1 bis 3 dargestellten Halterung 14 um 90° gedreht ist.

Das Atemtherapiegerät 10 kann mittels des Bandes 16 wie eine Schmuckkette oder ein Schmuckarmband um den Hals oder das Armgelenk eines Patienten gelegt werden. Insbesondere dann, wenn das Band 16 um den Hals gelegt wird, sollte die Länge des Bandes 16 so gewählt werden, dass das Therapieelement 12; 112 in den Mund genommen werden kann, ohne das Atemtherapiegerät 10 abzulegen.

Im Falle eines Atemnotanfalls kann der Patient das Therapieelement 12; 112 schnell und einfach greifen und hineinblasen, so dass ein Kollabieren der Atemwege verhindert werden kann.

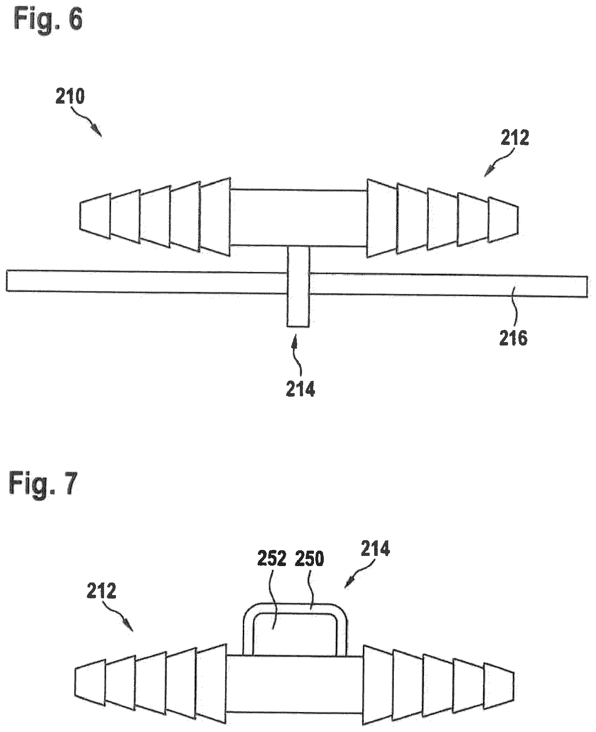

In den Fig. 5 bis 7 sind alternative Ausführungsformen eines Atemtherapiegeräts 210 gezeigt. Der Aufbau des Therapieelements 212 entspricht dem der in den Fig. 1 bis 4 dargestellten Ausführungsformen. Das Band 216 ist als Flachband ausgebildet und vorzugsweise lösbar mit der Halterung 214 verbunden. Auch hier können entsprechende Verschlüsse oder dergleichen vorgesehen sein. Als Halterung 214 ist ein eckiger Rahmen 250 mit einem Durchgang 252 vorgesehen, durch den das Band 216 geführt werden kann. Die Ausführungsformen der Fig. 5 und 6 unterscheiden sich von der Ausführungsform der Fig. 7 dadurch, dass die Halterung 214 bzw. der eckige Rahmen 252 um 90° gedreht ist.

Vorzugsweise ist die Länge des Bandes 216 einstellbar. Zusätzlich oder alternativ kann das Band 216 aus einem elastischen Material bestehen.

Die in den Fig. 5 bis 7 beschriebenen Ausführungsformen eines Atemtherapiegeräts 210 eignen sich besonders für das Tragen in Form eines Armbands am Handgelenk.

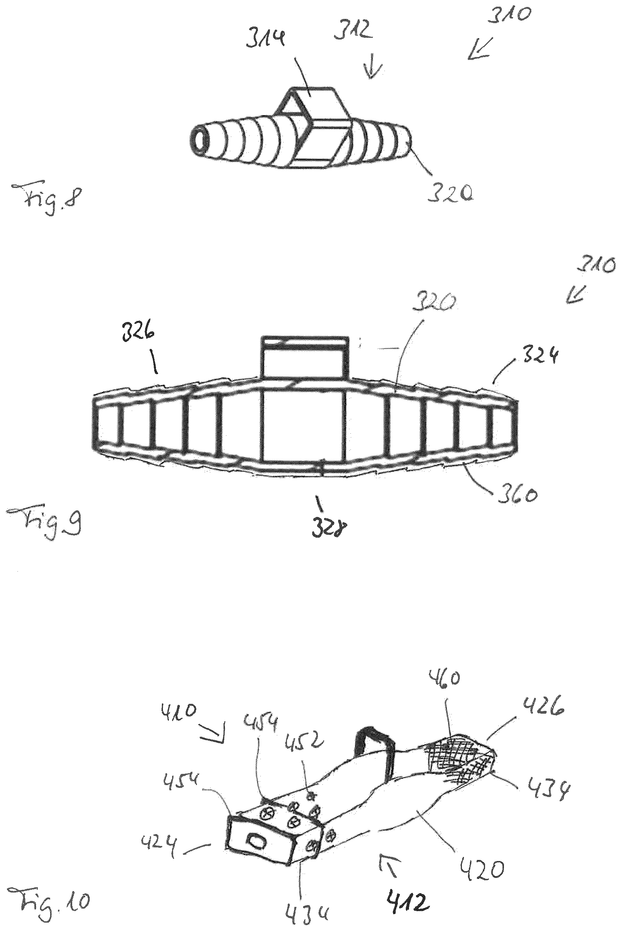

In den Figuren 8 und 9 ist eine vierte Ausführungsform eines Therapieelements 312 eines Atemtherapiegeräts 310 gezeigt. Das Therapieelement 312 unterscheidet sich im Wesentlichen von den bisher dargestellten Ausführungsformen dadurch, dass die Endabschnitte ohne Absenkung in den mittleren Abschnitt übergehen und die Halterung 314 massiver ausgestaltet ist.

Während bei der in den Figuren 1 bis 7 dargestellten Ausführungsformen die Länge des mittleren Abschnitts in etwa der Länge eines Endabschnitts entspricht, ist bei der in den Fig. 8 und 9 dargestellten Ausführungsform der mittlere Abschnitt 328 kürzer als ein Endabschnitt 324, 326. Des Weiteren unterscheidet sich die Ausführungsform dadurch, dass an der Außenseite des Therapieelements 312 eine Schicht 360 aus einem rutschfesten Material vorgesehen ist. Das rutschfeste Material besteht aus einem im Vergleich zum Grundkörper 320 weichen Material wie etwa einem weichen Silikonmaterial.

Eine fünfte Ausführungsform eines Atemtherapiegeräts 410 mit einem Therapieelement 412 ist in Fig. 10 gezeigt. Der Aufbau des nicht dargestellten Strömungskanals sowie der nicht dargestellten Strömungskammer entspricht denen der vorherigen Ausführungsformen.

Das an dem Grundkörper 420 angeformte Mundstück 434 weist eine Ober- und eine Unterseite mit jeweils einer Seitenbreite und einer Seitenlänge auf, wobei der Abstand zwischen der Oberund Unterseite deutlich geringer als die Seitenbreite der Ober- und der Unterseite ist. Insbesondere ist die Seitenbreite der Ober- und der Unterseite mindestens doppelt so groß wie der Abstand zwischen der Ober- und Unterseite. Hierdurch weist das Mundstück 434 einen rechteckigen Querschnitt mit abgerundeten Ecken auf. Die Form des Mundstückes 434 entspricht in etwa der eines Entenschnabels.

Die Außenseite des Grundkörpers 420 ist an dem ersten Endabschnitt 424 glatt ausgebildet. Auf der Ober- und der Unterseite sowie auf den Seitenwänden des Grundkörpers 420 befinden sich im Bereich des Mundstückes lokal begrenzte Silikonabschnitte 452, um die Rutschfestigkeit des Therapieelements 412 zu erhöhen. Zusätzlich sind umlaufende Rippen 454 vorgesehen, um das Mundstück 424 besser mit den Lippen greifen zu können. Die Außenseite des Grundkörpers 420 an dem zweiten Endabschnitt 426 ist vollflächig mit einer rutschfesten Materialschicht 460 aus Silikon überzogen.

Zusätzlich können auf der Unter und/oder Oberseite nicht dargestellte Mulden vorgesehen sein, um die Griffigkeit des Mundstücks 434 zu erhöhen.

Anstelle der Form eines Entenschnabels können die Mundstücke auch die Form einer Zigarettenspritze oder einer Trillerpfeife aufweisen.

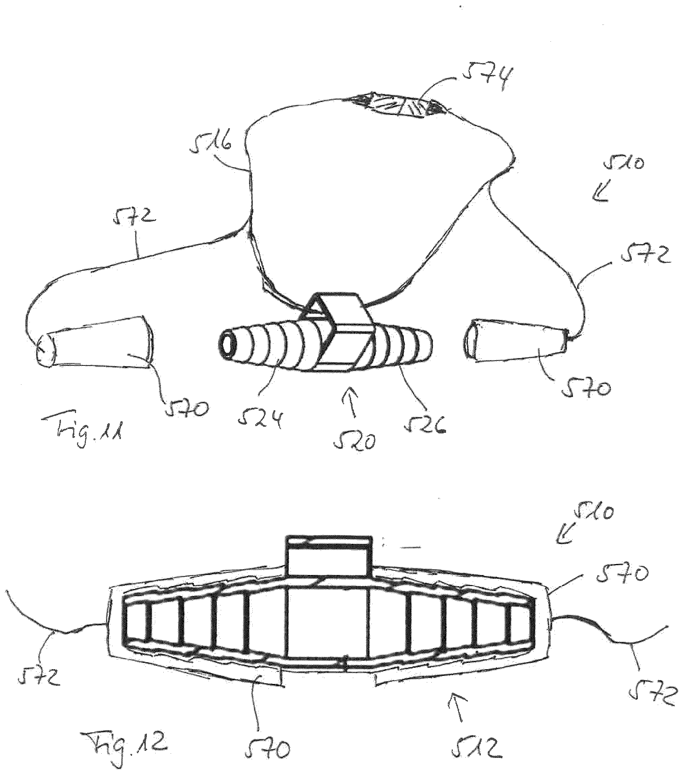

Die in den Fig. 11 und 12 dargestellte Ausführungsform zeigt ein Atemtherapiegerät 510 mit einer Schutzhülle 570. Die Schutzhülle 570 ist zweiteilig ausgebildet und kann jeweils auf einen Endabschnitt 524, 526 des Grundkörpers 520 aufgesteckt werden. Jeweils an den Enden befinden sich Belüftungsöffnungen, die für eine Belüftung des Strömungskanals des Therapieelements sorgen. Die beiden Schutzhüllenteile 570 sind mittels eines Bandes 572 oder einer Schnur mit einem Halsband 516 verbunden, so dass sie nicht verloren gehen, wenn sie von dem Atemtherapiegerät 510 abgezogen sind. Das Halsband 516 verfügt über einen Sicherheitsverschluss 574, der sich öffnet, falls zu fest an dem Halsband 516 gezogen wird, wenn ein Patient dieses trägt.

Bei nicht dargestellten Ausführungsformen kann die Schutzhülle einteilig ausgeführt sein, wobei sie eine Aussparung im Bereich der Halterung aufweist und bei gleicher Ausformung wie das Therapieelement durch Verwendung eines biegsamen Materials über den Grundkörper gestülpt und abgezogen werden kann. Alternativ kann eine weitere Ausführungsform einer einteiligen Hülle seitlich auf den Grundkörper aufgeschoben werden. Bei dieser Ausführungsform weist die Hülle eine zylindrische Form konstanten Durchmessers, geringfügig größer als der Durchmesser des mittleren Abschnitts des Therapieelements, auf und ist für die Aufnahme der Halterung auf einer Hälfte ausgeschnitten. Auch diese Ausführungsformen sind mittels einer Schnur oder eines Bandes mit dem Halsband verbunden und weisen zudem Belüftungsöffnungen an den Stirnseiten auf.

Das Band kann unmittelbar an der Halterung, beispielsweise mittels einer Schlaufe oder eines Knotens oder mittels eines weiteren Befestigungselements wie etwa eines Hakens, insbesondere eines Karabinerhakens an der Halterung befestigt sein. Anstelle eines Hakens können auch andere lösbare Verbindungen, beispielsweise in Form von Steckverbindungen vorgesehen sein. Wenn auch nicht dargestellt, so ist auch vorgesehen, dass der mittlere Abschnitt länger als ein Endabschnitt ist.

Bei nicht dargestellten Ausführungsformen ist die Strömungskammer 36 weggelassen. Zusätzlich oder alternativ ist bei einer weiteren Ausführungsform der Querschnitt des Strömungskanals im ersten Endabschnitt 24 und/oder zweiten Endabschnitt 26 konstant.

Die in den Figuren 1 bis 12 dargestellten Therapieelemente weisen einen zweiten Endabschnitt 26; 526 auf, der spiegelsymmetrisch zu dem ersten Endabschnitt 24; 524 ausgebildet ist. Die Länge und Form des Strömungskanals des ersten Endabschnitts 24; 524 und des zweiten Endabschnitts 26; 526 sind somit gleich ausgebildet. Im Rahmen der Erfindung kann der Strömungskanalabschnitt im ersten Endabschnitt 24; 524 unterschiedlich zu dem Strömungskanalabschnitt im zweiten Endabschnitt 26; 526 ausgebildet sein. Hierbei können beispielsweise die Länge, der Durchmesser oder die Form des Strömungskanals unterschiedlich sein.

Anstelle des Bandes können andere Befestigungsmittel gewählt werden, um das Therapieelement beispielsweise an der Bekleidung zu befestigen.

## Patentansprüche

1. Atemtherapiegerät umfassend ein Therapieelement (12; 112; 212; 312; 412; 512) und eine Halterung (14; 114; 214; 314), wobei das Therapieelement (12; 112; 212; 312; 412; 512) einen länglichen Grundkörper (20; 320; 420; 520) mit einem ersten Endabschnitt (24; 324; 424; 524) und einem zweiten Endabschnitt (26; 326; 426; 526), die entgegengesetzt zueinander angeordnet sind, sowie einen mittleren Abschnitt (28; 328) umfasst,
wobei ein Mundstück (34; 434) am ersten Endabschnitt (24; 324; 424; 524) vorgesehen ist,
wobei der Grundkörper (20; 320; 420; 520) eine erste Öffnung (30) an der Stirnseite des ersten Endabschnitts (24; 324; 424; 524) und eine zweite Öffnung (32) an der Stirnseite des zweiten Endabschnitts (26; 326; 426; 526) aufweist und im Inneren des Grundkörpers (20) ein Strömungskanal (22) vorgesehen ist, der sich von der ersten Öffnung (30) an der ersten Stirnseite bis zur zweiten Öffnung (32) an der zweiten Stirnseite erstreckt,
wobei an dem zweiten Endabschnitt (26; 326; 426; 526) ein Mundstück (34; 434) vorgesehen ist, wobei die Halterung (14; 114; 214; 314) an der Außenseite des Grundkörpers (20; 320; 420; 520), vorzugweise im Bereich des mittleren Abschnitts (28) vorgesehen ist, und **dadurch gekennzeichnet, dass** das Mundstück (34) am ersten Endabschnitt trichterförmig ausgebildet ist, wobei sich der Außendurchmesser des Mundstücks (34) am ersten Endabschnitt von der ersten Öffnung (30) in Richtung des mittleren Abschnitts (28; 328) erweitert.

2. Atemtherapiegerät nach Anspruch 1, **dadurch gekennzeichnet, dass** das Mundstück (34; 434) am ersten Endabschnitt einstückig mit dem Grundkörper (20) verbunden ist.

3. Atemtherapiegerät nach einem der vorhergehenden Ansprüche, **dadurch gekennzeichnet, dass** auf der Außenseite des Mundstücks (434) am ersten Endabschnitt wenigstens abschnittsweise eine rutschfeste Materialschicht (360; 452, 460) vorgesehen ist.

4. Atemtherapiegerät nach einem der vorhergehenden Ansprüche, **dadurch gekennzeichnet, dass** die Oberfläche des Mundstücks (34) am ersten Endabschnitt zumindest abschnittsweise uneben, insbesondere stufig ist.

5. Atemtherapiegerät nach einem der vorhergehenden Ansprüche, **dadurch gekennzeichnet, dass** das Mundstück (434) am ersten Endabschnitt wenigstens eine Vertiefung, insbesondere wenigstens eine Mulde und/oder wenigstens eine Erhöhung, insbesondere wenigstens eine Rippe (454) an seiner Außenseite aufweist.

6. Atemtherapiegerät nach einem der vorhergehenden Ansprüche, **dadurch gekennzeichnet, dass** der Grundkörper (20; 320; 420; 520) des Therapieelements (12; 112; 212; 312; 412; 512) aus einem festen formstabilen Material, insbesondere aus Kunststoff hergestellt ist.

7. Atemtherapiegerät nach einem der vorhergehenden Ansprüche, **dadurch gekennzeichnet, dass** sich der Strömungskanal (22) von der ersten Öffnung (30) zum mittleren Abschnitt (28) erweitert und sich vom mittleren Abschnitt (28) zur zweiten Öffnung (32) verengt, wobei sich insbesondere der Strömungskanal (22) von der ersten Öffnung (30) zum mittleren Abschnitt (28) konisch erweitert und sich vom mittleren Abschnitt (28) zur zweiten Öffnung (32) konisch verengt.

8. Atemtherapiegerät nach einem der Ansprüche 1 bis 6, **dadurch gekennzeichnet, dass** der Strömungskanal im Bereich des ersten Endabschnitts und/oder im Bereich des zweiten Endabschnitts einen konstanten Durchmesser aufweist, wobei vorzugsweise der Durchmesser des Strömungskanals konstant ist.

9. Atemtherapiegerät nach einem der vorhergehenden Ansprüche, **dadurch gekennzeichnet, dass** die Halterung (14; 114; 214; 314) mittig zwischen den beiden Endabschnitten (24, 26; 324, 326; 424, 426) angeordnet ist.

10. Atemtherapiegerät nach einem der vorhergehenden Ansprüche, **dadurch gekennzeichnet, dass** der erste Endabschnitt (24; 324; 424; 524) und der zweite Endabschnitt (26; 326; 426; 526) spiegelsymmetrisch zum mittleren Abschnitt (28) ausgebildet sind.

11. Atemtherapiegerät nach einem der vorhergehenden Ansprüche, **dadurch gekennzeichnet, dass** der Durchmesser des Strömungskanals (22) im Bereich von 2 mm bis 8 mm, vorzugsweise im Bereich von 3 mm bis 7 mm liegt.

12. Atemtherapiegerät nach einem der vorhergehenden Ansprüche, **dadurch gekennzeichnet, dass** die Länge des Strömungskanals (22) etwa 3 cm bis 7 cm, vorzugsweise 4 cm bis 6 cm beträgt.

13. Atemtherapiegerät nach einem der vorhergehenden Ansprüche, **dadurch gekennzeichnet, dass** mit dem Atemtherapiegerät ein positiver Ausatemdruck (PEP) beim Ausatmen in das Atemtherapiegerät erzeugbar ist, wobei der positive Ausatemdruck (PEP) kleiner gleich 10 mbar ist, vorzugsweise im Bereich von 5 mbar bis 1 mbar liegt.

14. Atemtherapiegerät nach einem der vorhergehenden Ansprüche, **dadurch gekennzeichnet, dass** die Halterung (14; 114; 214; 314) einen Durchgang (40; 250) und/oder ein Befestigungselement für ein Befestigungsmittel umfasst.

15. Atemtherapiegerät nach einem der vorhergehenden Ansprüche, **dadurch gekennzeichnet, dass** ein Band (16; 216; 516) vorgesehen ist, wobei das Band (16; 216; 516) an der Halterung (14; 114; 214; 314) befestigt ist.

16. Atemtherapiegerät nach einem der vorhergehenden Ansprüche, **dadurch gekennzeichnet, dass** eine Schutzhülle (570) vorgesehen ist, die das Therapieelement (512) wenigstens abschnittsweise umgibt.

## Claims

1. A respiratory therapy device comprising a therapy element (12; 112; 212; 312; 412; 512) and a holder (14; 114; 214; 314), wherein the therapy element (12; 112; 212; 312; 412; 512) comprises an elongated base body (20; 320; 420; 520) having a first end section (24; 324; 424; 524) and a second end section (26; 326; 426; 526) arranged opposite to each other, and a middle section (28; 328),
wherein a mouthpiece (34; 434) is provided at the first end section (24; 324; 424; 524),
wherein the base body (20; 320; 420; 520) has a first opening (30) at the front side of the first end section (24; 324; 424; 524) and a second opening (32) at the front side of the second end section (26; 326; 426; 526) and a flow channel (22) is provided within the base body (20) that extends from the first opening (30) at the first front side to the second opening (32) at the second front side, wherein at the second end section (26; 326; 426; 526) a mouthpiece (34; 343) is provided, wherein the holder (14; 114; 214; 314) is provided on the outside of the base body (20; 320; 420; 520), preferably in the region of the middle section (28), and is **characterized in that** the mouthpiece (34) is funnel-shaped at the first end section (34), wherein the outer diameter of the mouthpiece (34) at the first end section expands from the first opening (30) in direction of the middle section (28; 328).

2. The respiratory therapy device according to claim 1, **characterized in that** the mouthpiece (34; 434) is integrally connected to the base body (20) at the first end section.

3. The respiratory therapy device according to one of the preceding claims, **characterized in that** a slip-proof material layer (360; 452; 460) is provided at least in sections on the outside of the mouthpiece (34) at the first end section.

4. The respiratory therapy device according to one of the preceding claims, **characterized in that** the surface of the mouthpiece (34) is uneven, particularly stepped, at least in sections at the first end section.

5. The respiratory therapy device according to one of the preceding claims, **characterized in that** the mouthpiece (434) has at least one recess, particularly at least a depression and/or at least one elevation, particularly at least one rib (454) on its outer side at the first end section.

6. The respiratory therapy device according to one of the preceding claims, **characterized in that** the base body (20; 320; 40; 520) of the therapy element (12; 112; 212; 312; 412; 512) is made of a solid, dimensionally stable material, particularly of plastic.

7. The respiratory therapy device according to one of the preceding claims, **characterized in that** the flow channel (22) expands from the first opening (30) to the middle section (28) and narrows from the middle section (28) to the second opening (32), wherein particularly the flow channel (22) expands conically from the first opening (30) to the middle section (28) and narrows conically from the middle section (28) to the second opening (32).

8. The respiratory therapy device according to one of the claims 1 to 6, **characterized in that** the flow channel has a constant diameter in the region of the first end section and/or in the region of the second end section, wherein the diameter of the flow channel is preferably constant.

9. The respiratory therapy device according to one of the preceding claims, **characterized in that** the holder (14; 114; 214; 314) is arranged centrally between the two end sections (24, 26; 324, 326; 424, 426).

10. The respiratory therapy device according to one of the preceding claims, **characterized in that** the first end section (24; 324; 424; 524) and the second end section (26; 326; 426; 526) are mirror-symmetrical to the middle section (28).

11. The respiratory therapy device according to one of the preceding claims, **characterized in that** the diameter of the flow channel (22) is in the range of 2 mm to 8 mm, preferably in the range of 3 mm to 7 mm.

12. The respiratory therapy device according to one of the preceding claims, **characterized in that** the length of the flow channel (22) is approximately 3 cm to 7 cm, preferably 4 cm to 6 cm.

13. The respiratory therapy device according to one of the preceding claims, **characterized in that** a positive expiratory pressure (PEP) can be generated with the respiratory therapy device when exhaling, wherein the positive expiratory pressure (PEP) is less or equal 10 mbar, preferably in the range of 5 mbar to 1 mbar.

14. The respiratory therapy device according to one of the preceding claims **characterized in that** the holder (14; 114; 214; 314) comprises a passage (40; 250) and/or a fastening element for fastening means.

15. The respiratory therapy device according to one of the preceding claims, **characterized in that** a strap (16; 216; 516) is provided, wherein the strap (16; 216; 516) is attached to the holder (14; 114; 214; 314).

16. The respiratory therapy device according to one of the preceding claims, **characterized in that** a protective cover (570) is provided that encompasses the therapy element (512) at least in sections.

## Revendications

1. Appareil de thérapie respiratoire muni d'un élément thérapeutique (12 ; 112 ; 212 ; 312 ; 412 ; 512) et d'un élément de maintien (14 ; 114 ; 214 ; 314), ledit élément thérapeutique (12 ; 112 ; 212 ; 312 ; 412 ; 512) incluant un corps de base allongé (20 ; 320 ; 420 ; 520) qui présente un premier tronçon d'extrémité (24 ; 324 ; 424 ; 524) et un second tronçon d'extrémité (26 ; 326 ; 426 ; 526) disposés en sens inverse l'un de l'autre, ainsi qu'un tronçon médian (28 ; 328),
un embout buccal (34 ; 434) étant prévu sur le premier tronçon d'extrémité (24 ; 324 ; 424 ; 524),
sachant que le corps de base (20 ; 320 ; 420 ; 520) comporte un premier orifice (30) à la face extrême dudit premier tronçon d'extrémité (24 ; 324 ; 424 ; 524) et un second orifice (32) à la face extrême du second tronçon d'extrémité (26 ; 326 ; 426 ; 526), et qu'il est prévu, dans l'espace interne du corps de base (20), un canal d'écoulement (22) s'étendant du premier orifice (30), situé à la première face extrême, jusqu'au second orifice (32) situé à la seconde face extrême,
un embout buccal (34 ; 434) étant prévu sur le second tronçon d'extrémité (26 ; 326 ; 426 ; 526), sachant que
l'élément de maintien (14 ; 114 ; 214 ; 314) est prévu à la face extérieure dudit corps de base (20 ; 320 ; 420 ; 520), de préférence dans la région du tronçon médian (28), **caractérisé par le fait**
**que** l'embout buccal (34) situé sur le premier tronçon d'extrémité est en forme d'entonnoir, le diamètre extérieur dudit embout buccal (34) au niveau du premier tronçon d'extrémité s'élargissant alors à partir du premier orifice (30) en direction du tronçon médian (28 ; 328).

2. Appareil de thérapie respiratoire selon la revendication 1, **caractérisé par le fait que** l'embout buccal (34 ; 434) situé sur le premier tronçon d'extrémité est relié d'un seul tenant au corps de base (20).

3. Appareil de thérapie respiratoire selon l'une des revendications précédentes, **caractérisé par le fait qu'**une couche (360 ; 452, 460) de matériau antidérapant est prévue au niveau du premier tronçon d'extrémité, au moins par zones, sur la face extérieure de l'embout buccal (434).

4. Appareil de thérapie respiratoire selon l'une des revendications précédentes, **caractérisé par le fait que** la surface de l'embout buccal (34) situé sur le premier tronçon d'extrémité est au moins partiellement inégale, en particulier en gradins.

5. Appareil de thérapie respiratoire selon l'une des revendications précédentes, **caractérisé par le fait que** l'embout buccal (434) situé sur le premier tronçon d'extrémité est doté d'au moins un renfoncement, en particulier d'au moins une augette, et/ou d'au moins une région éminente, en particulier d'au moins une nervure (454) à sa face extérieure.

6. Appareil de thérapie respiratoire selon l'une des revendications précédentes, **caractérisé par le fait que** le corps de base (20 ; 320 ; 420 ; 520) de l'élément thérapeutique (12 ; 112 ; 212 ; 312 ; 412 ; 512) est fabriqué en un matériau solide doué de stabilité de forme, notamment en matière plastique.

7. Appareil de thérapie respiratoire selon l'une des revendications précédentes, **caractérisé par le fait que** le canal d'écoulement (22) s'élargit vers le tronçon médian (28), à partir du premier orifice (30), et se rétrécit vers le second orifice (32) à partir dudit tronçon médian (28),
sachant notamment que ledit canal d'écoulement (22) présente un élargissement tronconique vers ledit tronçon médian (28), à partir dudit premier orifice (30), et un rétrécissement tronconique vers le second orifice (32) à partir dudit tronçon médian (28).

8. Appareil de thérapie respiratoire selon l'une des revendications 1 à 6, **caractérisé par le fait que** le canal d'écoulement possède un diamètre constant dans la région du premier tronçon d'extrémité et/ou dans la région du second tronçon d'extrémité, ledit diamètre du canal d'écoulement étant préférentiellement constant.

9. Appareil de thérapie respiratoire selon l'une des revendications précédentes, **caractérisé par le fait que** l'élément de maintien (14 ; 114 ; 214 ; 314) occupe un emplacement central entre les deux tronçons d'extrémité (24, 26 ; 324, 326 ; 424, 426).

10. Appareil de thérapie respiratoire selon l'une des revendications précédentes, **caractérisé par le fait que** le premier tronçon d'extrémité (24 ; 324 ; 424 ; 524) et le second tronçon d'extrémité (26 ; 326 ; 426 ; 526) sont réalisés avec symétrie spéculaire par rapport au tronçon médian (28).

11. Appareil de thérapie respiratoire selon l'une des revendications précédentes, **caractérisé par le fait que** le diamètre du canal d'écoulement (22) se situe dans la plage de 2 mm à 8 mm, préférentiellement dans la plage de 3 mm à 7 mm.

12. Appareil de thérapie respiratoire selon l'une des revendications précédentes, **caractérisé par le fait que** la longueur du canal d'écoulement (22) mesure d'environ 3 cm à 7 cm, préférentiellement de 4 cm à 6 cm.

13. Appareil de thérapie respiratoire selon l'une des revendications précédentes, **caractérisé par le fait que** ledit appareil de thérapie respiratoire permet d'engendrer une pression expiratoire positive (PEP) lors de l'expiration dans ledit appareil de thérapie respiratoire, laquelle pression expiratoire positive (PEP) est inférieure ou égale à 10 mbar et se situe, de préférence, dans la plage de 5 mbar à 1 mbar.

14. Appareil de thérapie respiratoire selon l'une des revendications précédentes, **caractérisé par le fait que** l'élément de maintien (14 ; 114 ; 214 ; 314) comprend un passage (40 ; 250) et/ou un élément de fixation dévolu à un moyen de fixation.

15. Appareil de thérapie respiratoire selon l'une des revendications précédentes, **caractérisé par le fait qu'**il est prévu une dragonne (16 ; 216 ; 516), laquelle dragonne (16 ; 216 ; 516) est fixée à l'élément de maintien (14 ; 114 ; 214 ; 314).

16. Appareil de thérapie respiratoire selon l'une des revendications précédentes, **caractérisé par le fait qu'**il est prévu une enveloppe protectrice (570) qui entoure, au moins par zones, l'élément thérapeutique (512).
